# EUROPEAN PATENT APPLICATION

(11) **EP 1 308 156 A1**
(43) Date of publication of application: **07.05.2003**
(21) Application number: 01955631.5
(22) Date of filing: 09.08.2001
(51) Int. Cl.: A61K 9/14, A61K 9/16, A61K 9/20, A61K 45/00, A61K 47/04, A61K 47/30, A61K 47/32, A61K 47/34, A61K 47/36, A61K 47/38

(54) **DRUG-CONTAINING SOLID DISPERSION HAVING IMPROVED SOLUBILITY**

(30) Priority: 11.08.2000 JP 2000243913
(71) Applicant: Eisai Co., Ltd., Tokyo 112-8088 (JP)
(72) Inventor: AOKI, Shigeru, Hashima-gun, Gifu 501-6027 (JP)
(74) Representative: HOFFMANN EITLE
(86) International application number: JP0106868
(87) International publication number: WO02013792

(57) **Abstract**

The present invention provides a solid dispersion composition comprising a slightly soluble medicament and having an improved solubility, which can be produced by a simple process with a low cost and is excellent in safety, and a process for producing it. It provides a solid dispersion composition, comprising a slightly soluble medicament blended and a water-soluble polymer, exposed to microwaves.

## Description

### Technical Field

The present invention relates to a solid dispersion composition having an improved solubility, which is exposed to microwaves, and to a process for producing it.

### Prior Art

In general, one of the most important factors affecting digestive absorption of drugs is solubility of the drugs. It is considered that slight solubility of a material drug is likely to retard the time for reaching an effective blood level, to reduce a bioavailability, and to vary the time and the bioavailability widely.

Various techniques for increasing the solubility of drugs have been known, and are broadly categorized into the following three: (1) increasing the specific surface area of the drug particle; (2) using amorphous substances or metastable crystals; and (3) using various salts or adding solubilizer. As the specific techniques, the followings are known. In technique (1), drugs are finely powdered, allowed to form solvates, or adsorbed on the surface of a carrier. In technique (2), crystal polymorph are used, mixing and pulverization is performed, or solid dispersions are prepared. In technique (3), acid salts or alkali salts are prepared, or pH buffers and/or surfactants are added.

In particular, solid dispersions have been of interest to improve the solubility of slightly soluble medicament prepared by a relatively simple method. As such production process, an organic solvent method, a heat-melting method, and a twin screw extruder method (disclosed in, for example, Japanese Patent No. 2527107 and JP-A 9-3098283) have widely been used. In the organic solvent method, however, it is required that a large amount of organic solvent be safely recovered, from the viewpoint of environmental conservation. This increases manufacturing cost and also brings about problems in terms of personnel health and safety. Also, in the heat-melting method and the twin screw extruder method, heat treatment liable to cause drugs to decompose or stain and, therefore, the available drugs are limited. In addition, these methods need complicated processes, such as pulverization, mixing and forming, after a solid dispersion has been prepared.

Accordingly, a solid dispersion capable of being prepared by simple processes with a low cost and ensuring environmental and personnel safety and a method for preparing it are desired.

According to a solid dispersion composition containing a slightly soluble medicament and having an improved solubility, development of a production process which is simple and low-cost process, and ensures environmental and personnel safety is earnestly desired.

### Disclosure of Invention

Considering the above-described circumstances, the inventors of the present invention have conducted intense research to achieve a solid dispersion composition containing a slightly soluble medicament and having an improved solubility, and a method for producing it. As a result, they have found that the following composition can lead to accomplishment of the desired object and have completed the present invention.

The present invention is a solid dispersion composition, comprising a slightly soluble medicament blended and a water-soluble polymer, exposed to microwaves. The slightly soluble medicament of the present invention refers to a drug hardly soluble in water. The solubility is not particularly limited, but a drug having a solubility of 0.05 mg/mL or less in water at 25°C is particularly effective. As the slightly soluble medicament, for example, nifedipine, phenytoin, nitrofurantoin, benoxaprofen, griseofulvin, sulfathiazole, tacrolimus, piroxicam, carbamazepine, phenacetin and cyclic GMP phosphodiesterase inhibitors may be proposed. However, it is needless to say that they are not limited to these compounds. The composition of the present invention is not particularly limited, and for example, it may be tablets, granules, fine granules or a powder.

Also, the present invention is a solid dispersion composition, comprising a slightly soluble medicament, a water-soluble polymer and silicic acid, exposed to microwaves.

Further, the present invention is a solid dispersion composition, comprising a slightly soluble medicament, 1) a water-soluble polymer or a water-soluble polymer and silicic acid and 2) water, exposed to microwaves.

Microwave exposure is a method in which microwaves vibrate water in a substance to heat the substance locally, and according to the present invention, the slightly soluble medicament and/or the water-soluble polymer can be melted, and thus, a solid dispersion can be extremely easily produced. Therefore, it is essential that the composition to be exposed to microwaves contain water. Preferably, water is added to the composition to be exposed to microwaves when required. The water content in the composition to be exposed to microwaves is generally in the range of 0.1 to 50% by weight or 1 to 50% by weight, preferably in the range of 0.5 to 40% by weight and further preferably in the range of 0.8% to 30% by weight.

If the composition to be exposed the microwaves contains water in too large amount to ensure hardness and formability sufficiently, a drying step may further be added. The drying step is not particularly limited. It may be dried by, for example, rack-drying in draft or hot air, or may be dried by using a fluidized bed. Drying temperature is generally in the range of 15 to 80°C, preferably in the range of 20 to 75°C and further preferably in the range of 30 to 70°C.

The silicic acid, which is added, if necessary, into the slightly soluble medicament in combination with the water-soluble polymer not only serves as a disintegrating agent, but also serves to hold water in the solid dispersion composition which may be tablets, granules, fine granules or a powder. In addition, it has the function of holding the shape of the composition such as tablets, granules, fine granules or a powder.

The composition comprising a slightly soluble medicament blended with a water-soluble polymer, or a water-soluble polymer and silicic acid, and further, if necessary, water may be prepared by, for example, mixing the slightly soluble medicament and these additives in a powder form and then tabletting to give a tablet. Or, it may be prepared by mixing, conducting dry-granulation or wet-granulation and drying, to give a granule, a fine granule or a powder, or tabletted to give a tablet. The dry-granulation is performed by using, for example, a roller compactor. The wet-granulation is performed by using, for example, a fluidized bed granulator, a tumbling granulator, an extruding granulator or a spray dryer.

Also, the present invention is a process for producing a solid dispersion composition of a slightly soluble medicament having an improved solubility, which comprises exposing a composition comprising a slightly soluble medicament blended and a water-soluble polymer or a water-soluble polymer and silicic acid to microwaves.

Further, the present invention is a process for producing a solid dispersion composition of a slightly soluble medicament having an improved solubility, which comprises exposing a composition comprising a slightly soluble medicament, 1) a water-soluble polymer or a water-soluble polymer and silicic acid and 2) water to microwaves.

In the present invention, the compounding ratio of the slightly soluble medicament is not particularly limited, but it is generally in the range of 0.1 to 50% by weight, preferably in the range of 0.1 to 30% by weight and further preferably in the range of 0.1 to 20% by weight, to the weight of the solid dispersion composition.

The average particle diameter of the raw slightly soluble medicament is in the range of 1 to 100 µm, preferably in the range of 1 to 70 µm and further preferably in the range of 1 to 50 µm.

As the water-soluble polymer, hydroxypropylmethyl cellulose, methylcellulose, hydroxypropyl cellulose, hydroxypropylmethyl cellulose phthalate, hydroxypropylmethyl cellulose acetate succinate, sodium carboxymethylcellulose, cellulose acetate succinate, agar, gelatin, sodium alginate, polyvinylpyrrolidone, aminoalkyl methacrylate copolymer, methacrylate copolymer, carboxyvinyl polymer, polyvinyl alcohol, macrogol etc. may be proposed. In the present invention, these may be used singly or in combination. The average particle diameter of the water-soluble polymer and additives which may be further added, is not particularly limited, but it is generally in the range of 0.1 to 400 µm, preferably in the range of 0.1 to 200 µm and further preferably in the range of 0.1 to 100 µm.

Further, as the compounding ratio of the slightly soluble medicament and water-soluble polymer in the present invention, the water-soluble polymer is compounded generally in the range of 0.5 to 10 parts by weight, preferably in the range of 1 to 8 parts by weight and further preferably in the range of 2 to 6 parts by weight, to 1 part by weight of the slightly soluble medicament.

A generally used disintegrating agent may be added to the solid dispersion composition of the present invention, if necessary.

As the disintegrating agents, for example, crystal cellulose, crospovidone, low substituted hydroxypropyl cellulose, sodium croscarmellose, calcium silicate, magnesium aluminometasilicate, carboxymethyl cellulose, calcium carboxymethyl cellulose, hydroxypropyl starch, sodium carboxymethyl starch, partially-gelatinized starch, sodium alginate etc. may be proposed in addition to silicic acid anhydride. In the present invention, these may be used singly or in combination.

In the present invention, the tablet formed of the solid dispersion composition may further contain a generally used lubricant, sweetening agent, colorant etc., if necessary.

As the lubricant, for example, magnesium stearate, calcium stearate, stearic acid and talc may be proposed.

As the sweetening agent, for example, aspartame, dipotassium glycyrrhizinate, sucrose, licorice, saccharin and sodium saccharine may be proposed. As the coloring agent, for example, yellow iron sesquioxide, yellow iron oxide, Food Yellow No. 4, Food Yellow No. 5, Food Yellow No. 4 aluminum lake, bengala, iron sesquioxide, Food red No. 2, Food Red No. 3 and Food Red NO. 102. In the present invention, these additives may be used singly or in combination.

In the present invention, the water content in the powder before exposing to microwaves is generally in the range of 0.1 to 40% by weight, preferably in the range of 0.1 to 35% by weight, and further preferably in the range of 0.1 to 30% by weight.

The tablet having an improved solubility of the present invention may be prepared, for example, as in the following procedure.

To 500 g of nifedipine which is a slightly soluble medicament are added 2500 g of grained macrogol 6000, 3000 g of light silicic acid anhydride and 1000 g of purified water, followed by mixing sufficiently. The mixture is placed in a mortar having a diameter of 10 mm, tabletted at a compression pressure of 100 kgf by using a compression tester (Autograph manufactured by Shimadzu), to prepare a tablet of 600 mg containing 50 mg of nifedipine. One tablet is placed in a 50 mL glass jar, and the opening of the glass jar is covered with a polyvinylidene chloride film having a small hole. Then, the tablet is exposed to microwaves for 3 or 4 minutes with a microwave generator (MDS-2000 manufactured by CEM Corporation, power: 630 W). After the exposure, the polyvinylidene chloride film is removed and the tablet is dried at 40°C for 18 hours, to give a tablet of 600 mg containing 50 mg of nifedipine having an improved solubility of nifedipine.

### Brief Description of the Drawings

Fig. 1 is a graph showing time-lapse changes of the amount of nifedipine dissolved from the tablet prepared as in Example 4 varying the microwave exposure time (0 to 4 minutes) into 500 ml of purified water (the paddle method at 50 rpm).
Fig. 2 is a graph showing the amount (the paddle method at 50 rpm) of the tablets of Examples 1, 4, 5 and 6 (microwave exposure time: 3 or 4 minutes) dissolved into 500 ml of purified water when 30 minutes have elapsed after the dissolution is started.

According to the present invention, by using a slightly soluble medicament, a solid dispersion composition having an improved solubility of the medicament can be prepared. Examples showing the effects of the invention will be shown below.

### Experimental Example

### Effect of microwave exposure

Tablets of Examples 1 to 6 shown below (slightly soluble medicament: nifedipine, microwave exposure time: 3 or 4 minutes) were subjected to evaluations by powder X-ray diffraction. For references, tablets not exposed to microwaves were prepared as the same formulae as in the respective Examples, and were subjected to the same experiment. The evaluation by powder X-ray diffraction was performed for powders prepared by lightly pulverizing the tablets in an agate mortar with an apparatus manufactured by Rigaku Industrial Corporation (INT-2500 Ultrax 18) under the following conditions.
- Employed X ray:: Cu K alpha ray
- Counter:: scintillation counter
- Goniometer:: vertical goniometer (RINT 2000)
- Applied voltage:: 40 kV
- Applied current:: 20 mA
- Scanning rate:: 2°/min
- Scanning axis:: 2θ
- Scanning range:: 2θ=5° to 30°
- Diverging slit:: 1°
- Scattering slit:: 1°
- Photo acceptance slit:: 0.15 mm

Furthermore, the disintegration time (in purified water) and hardness of the tablets were measured in accordance with the disintegration test and the hardness test specified in the Japanese Pharmacopoeia.

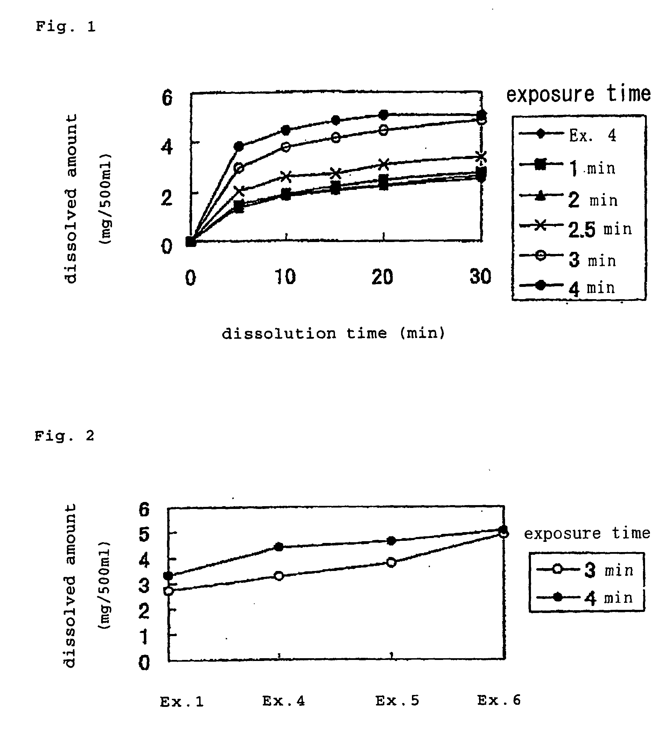

Also, powder X-ray diffraction and a dissolution test were performed for tablets prepared in an identical manner to Example 4 shown below, except that the microwave exposure times were varied to 0 (not exposed), 1, 2, 2.5, 3, and 4 minutes. The dissolution test was performed for one tablet in 50 mL of purified water by the paddle method (50 rpm) in accordance with the dissolution test specified in the Japanese Pharmacopoeia. After 5, 10, 15, 20 and 30 minutes had elapsed, samples were taken and the dissolution amount of nifedipine with time were measured by ultraviolet absorption spectrophotometry (measurement wavelength: two wavelengths of 350 and 450 nm). The prescriptions of Examples 1 to 6 are shown in Table 1. The results of the dissolution test are shown in Fig. 1.

**Table 1**

| | Examples | | | | | |
|---|---|---|---|---|---|---|
| | 1 | 2 | 3 | 4 | 5 | 6 |
| niphedipine | 100 | 100 | 100 | 100 | 100 | 100 |
| macrogol 6000 | 500 | 500 | 500 | 500 | 500 | 500 |
| light anhydrous silicic acid | 600 | 400 | 200 | 600 | 600 | 600 |
| water | - | - | - | 200 | 400 | 600 |
| tablet weight | 1200 | 1000 | 800 | 1200 | 1200 | 1200 |
| (Unit: mg) | | | | | | |

As a result of the evaluation by the powder X-ray diffraction, the tablets of Examples 1 to 6 did not exhibit diffraction peaks originating from nifedipine crystals in all the tablets and were amorphous. In contrast, all of the reference samples of the respective Examples exhibited diffraction peaks originating from nifedipine crystals. There were no significant difference in the hardness and disintegration time of the tablets between Examples and Reference Examples.

As shown in Fig. 1, the tablets containing nifedipine prepared by exposing microwaves for 2 minutes or less exhibited no difference in dissolution with time from the tablet not exposed to the microwave. However, the tablets exposed to the microwave for 2.5 minutes or more exhibited increase in the dissolution rate. Specifically, as exposure time was increased, the dissolution rate increased. In the evaluation by the powder X-ray diffraction, the tablets exposed to the microwaves for 2 minutes or less exhibited the diffraction peaks originating from nifedipine crystals, and the peaks disappeared by exposing for 2.5 minutes or more.

Accordingly, it is evident that, by exposing microwaves in the present invention, a solid dispersion tablet containing amorphous nifedipine can be obtained and that the solid dispersion composition shows an excellent solubility.

### Effect of water added in the present invention

Tablets of Examples 1 and 4 to 6 shown below (slightly soluble medicament: nifedipine, microwave exposure time: 3 and 4 minutes) were subjected to dissolution test. The prescriptions of additives for these tablets were completely the same, except for the amount of purified water added to the composition before exposing to microwaves. The amount of purified water added in Examples 1, 4, 5, and 6 were 0% (not added), 16.7%, 33.3% and 50% by weight, respectively. The dissolution test was performed for one tablet in 50 mL of purified water by the paddle method (50 rpm) in accordance with the dissolution test specified in the Japanese Pharmacopoeia. The sample solutions were taken after 30 minutes had elapsed, and the dissolution amount of nifedipine (after a lapse of 30 minutes) was measured by ultraviolet absorption spectrophotometry (measurement wavelength: two wavelengths of 350 and 450 nm).

The results of the dissolution test are shown in Fig. 2.

As shown in Fig. 2, it is recognized that as the amount of purified water added was increased, the amount of nifedipine dissoved (after a lapse of 30 minutes) from the solid dispersion tablets containing nifedipine increased, in comparison with that from the tablet to which purified water was not added (Example 1). In the powder X-ray diffraction, the disappearance of the diffraction peak originating from nifedipine crystals was observed in all the samples.

In the solid dispersion composition of the present invention, it is evident that the higher the water content in the composition to be exposed to microwaves is, the higher the effect of dissolving a slightly soluble material and/or a water-soluble polymer by microwaves is, and that the solid dispersion composition shows an excellent solubility.

### Examples

Hereinafter, the present invention will be illustrated in more detail with reference to Examples, but it is not limited to these.

### Example 1

In a mixer, 100 g of nifedipine, 500 g of grained macrogol 6000 and 600 g of light silicic acid anhydride were sufficiently mixed. The mixture was placed in a mortar having a diameter of 20 mm, tabletted at a compression pressure of 100 kgf by using a compression tester (Autograph manufactured by Shimadzu), to prepare a tablet of 1200 mg containing 100 mg of nifedipine. One tablet was placed in a 50 mL glass jar, and the opening of the glass jar was covered with a polyvinylidene chloride film having a small hole. Then, the tablet was exposed to microwaves for 3 or 4 minutes with a microwave generator (MDS-2000 manufactured by CEM Corporation, power: 630 W), to give a tablet having an improved solubility.

### Example 2

In a mixer, 100 g of nifedipine, 500 g of grained macrogol 6000 and 400 g of light silicic acid anhydride were sufficiently mixed. The mixture was placed in a mortar having a diameter of 20 mm, tabletted at a compression pressure of 100 kgf by using a compression tester (Autograph manufactured by Shimadzu), to prepare a tablet of 1000 mg containing 100 mg of nifedipine. One tablet was placed in a 50 mL glass jar, and the opening of the glass jar was covered with a polyvinylidene chloride film having a small hole. Then, the tablet was exposed to microwaves for 3 or 4 minutes with a microwave generator (MDS-2000 manufactured by CEM Corporation, power: 630 W), to give a tablet having an improved solubility.

### Example 3

In a mixer, 100 g of nifedipine, 500 g of grained macrogol 6000 and 200 g of light silicic acid anhydride were sufficiently mixed. The mixture was placed in a mortar having a diameter of 20 mm, tabletted at a compression pressure of 100 kgf by using a compression tester (Autograph manufactured by Shimadzu), to prepare a tablet of 800 mg containing 100 mg of nifedipine. One tablet was placed in a 50 mL glass jar, and the opening of the glass jar was covered with a polyvinylidene chloride film having a small hole. Then, the tablet was exposed to microwaves for 3 or 4 minutes with a microwave generator (MDS-2000 manufactured by CEM Corporation, power: 630 W), to give a tablet having an improved solubility.

### Example 4

In a mixer, 100 g of nifedipine, 500 g of grained macrogol 6000 and 600 g of light silicic acid anhydride were lightly mixed. Further, 200 g of purified water was added thereto, followed by mixing sufficiently. The mixture was placed in a mortar having a diameter of 20 mm, tabletted at a compression pressure of 100 kgf by using a compression tester (Autograph manufactured by Shimadzu), to prepare a tablet of 1200 mg containing 100 mg of nifedipine. One tablet was placed in a 50 mL glass jar, and the opening of the glass jar was covered with a polyvinylidene chloride film having a small hole. Then, the tablet was exposed to microwaves for 3 or 4 minutes with a microwave generator (MDS-2000 manufactured by CEM Corporation, power: 630 W). After the exposure, the polyvinylidene chloride film was removed and the tablet was dried at 40°C for 18 hours, to give a tablet having an improved solubility.

### Example 5

In a mixer, 100 g of nifedipine, 500 g of grained macrogol 6000 and 600 g of light silicic acid anhydride were lightly mixed. Further, 400 g of purified water was added thereto, followed by mixing sufficiently. The mixture was placed in a mortar having a diameter of 20 mm, tabletted at a compression pressure of 100 kgf by using a compression tester (Autograph manufactured by Shimadzu), to prepare a tablet of 1200 mg containing 100 mg of nifedipine. One tablet was placed in a 50 mL glass jar, and the opening of the glass jar was covered with a polyvinylidene chloride film having a small hole. Then, the tablet was exposed to microwaves for 3 or 4 minutes with a microwave generator (MDS-2000 manufactured by CEM Corporation, power: 630 W). After the exposure, the polyvinylidene chloride film was removed and the tablet was dried at 40°C for 18 hours, to give a tablet having an improved solubility.

### Example 6

In a mixer, 100 g of nifedipine, 500 g of grained macrogol 6000 and 600 g of light silicic acid anhydride were lightly mixed. Further, 600 g of purified water was added thereto, followed by mixing sufficiently. The mixture was placed in a mortar having a diameter of 20 mm, tabletted at a compression pressure of 100 kgf by using a compression tester (Autograph manufactured by Shimadzu), to prepare a tablet of 1200 mg containing 100 mg of nifedipine. One tablet was placed in a 50 mL glass jar, and the opening of the glass jar was covered with a polyvinylidene chloride film having a small hole. Then, the tablet was exposed to microwaves for 3 or 4 minutes with a microwave generator (MDS-2000 manufactured by CEM Corporation, power: 630 W). After the exposure, the polyvinylidene chloride film was removed and the tablet was dried at 40°C for 18 hours, to give a tablet having an improved solubility.

## Claims

1. A solid dispersion composition, comprising a slightly soluble medicament blended and a water-soluble polymer, exposed to microwaves.

2. A solid dispersion composition, comprising a slightly soluble medicament, a water-soluble polymer and silicic acid, exposed to microwaves.

3. A solid dispersion composition, comprising a slightly soluble medicament, 1) a water-soluble polymer or a water-soluble polymer and silicic acid and 2) water, exposed to microwaves.

4. A solid dispersion composition, comprising a slightly soluble medicament, 1) a water-soluble polymer or a water-soluble polymer and silicic acid and 2) water, exposed to microwaves, then dried.

5. The solid dispersion composition according to any of Claims 1 to 4, wherein the water content in the composition to be exposed to microwaves is in the range of 0.1 to 50 % by weight.

6. The solid dispersion composition according to any of Claims 1 to 4, wherein the water content in the composition to be exposed to microwaves is in the range of 0.1 to 40% by weight.

7. The solid dispersion composition according to any of Claims 1 to 6, wherein the solid dispersion composition is tablets, granules, fine granules or a powder.

8. The solid dispersion composition according to any of Claims 1 to 7, wherein the solubility of the slightly soluble medicament in water at 25°C is 0.05 mg/mL or less.

9. A process for producing a solid dispersion composition of a slightly soluble medicament having an improved solubility, which comprises exposing a composition comprising a slightly soluble medicament blended and a water-soluble polymer or a water-soluble polymer and silicic acid to microwaves.

10. A process for producing a solid dispersion composition of a slightly soluble medicament having an improved solubility, which comprises exposing a composition comprising a slightly soluble medicament, 1) a water-soluble polymer or a water-soluble polymer and silicic acid and 2) water to microwaves.
